# EUROPEAN PATENT SPECIFICATION

(11) **EP 4 247 335 B1**
(45) Date of publication and mention of the grant of the patent: **12.02.2025**
(21) Application number: 21815896.2
(22) Date of filing: 17.11.2021
(51) Int. Cl.: A61K 9/02, A61K 47/36, A61P 1/00, A61K 31/05, A61K 31/728, A61P 15/02

(54) **PHARMACEUTICAL COMPOSITION COMPRISING CANNABIDIOL AND HYALURONIC ACID**
PHARMAZEUTISCHE ZUSAMMENSETZUNG MIT CANNABIDIOL UND HYALURONSÄURE
COMPOSITION PHARMACEUTIQUE COMPRENANT DU CANNABIDIOL ET DE L'ACIDE HYALURONIQUE

(30) Priority: 19.11.2020 CZ 20200620
(43) Date of publication of application: 27.09.2023
(73) Proprietor: CB21 Pharma, S.R.O., 61200 Brno (CZ)
(72) Inventor: STORCH, Jan, 10000 Praha 10 (CZ); PRUSOVA, Lenka, 27201 Kladno (CZ)
(74) Representative: Harber IP s.r.o.
(86) International application number: PCT/CZ2021/050136
(87) International publication number: WO 2022/105952

(56) References cited:
- US-A1- 2018 110 753
- SCHOONEN A J M ET AL: "Release of drugs from fatty suppository bases I. The release mechanism", INTERNATIONAL JOURNAL OF PHARMACEUTICS, ELSEVIER, NL, vol. 4, no. 2, 1 December 1979 (1979-12-01), pages 141 - 152, XP023844917, ISSN: 0378-5173, [retrieved on 19791201], DOI: 10.1016/0378-5173(79)90062-0
- DE VILLIERS MELGARDT: "Suppository Bases", 19 October 2008 (2008-10-19), Internet, XP055884435, Retrieved from the Internet <URL:https://www.researchgate.net/publication/318380307_Suppository_Bases#:~:text=Base%201%20is%20a%20good,%2C%20water%2Dsoluble%20suppository%20base.> [retrieved on 20220127]
- "Handbook of Pharmaceutical Excipients", 2 January 2003, PHARMACEUTICAL PRESS, ISBN: 978-0-85369-472-4, article UNKNOWN: "Suppository Bases, Hard Fat", pages: 636 - 640, XP055311179

## Description

### Field of Art

The present invention relates to a pharmaceutical composition containing cannabidiol and hyaluronic acid or a salt thereof with an improved dissolution profile and an improved anti-inflammatory action in the form of suppositories.

### Background Art

Tissues of the anorectal canal and anus can be damaged by cracks or fissures in the mucosa in various diseases or problems. These cracks and fissures are manifested by pain during or after defecation, bleeding, burning and itching of the rectum. They can also easily become infected with intestinal bacteria, resulting in a non-healing ulcer. Similarly, damage to vaginal mucosa can cause painful conditions, bleeding, act as a gateway for bacteria, viruses or fungi to enter the body, and lead to vaginal inflammation.

These conditions can be improved by ensuring adequate hydration of the affected tissues. Sodium hyaluronate suppository treatments can be used for this. For example, commercially available Cicatridina vaginal suppositories and Proktis-M rectal suppositories contain sodium hyaluronate and extracts from *Centella asiatica,* marigold, aloe vera, and tea tree extract or essential oil.

Despite significant efforts to standardize them, plant extracts have variable compositions. Furthermore, the suppositories contain a mixture of plant extracts or essential oils, some of which may contain mucosal irritants. Furthermore, it is necessary to enhance the healing properties of such a preparation and to eliminate undesirable inflammatory conditions.

A phytoceutic for which there is still little clinical data on its effect on pelvic tissues is cannabidiol (CBD), a non-psychotropic major compound of a complex extract of plants of the genus *Cannabis.* CBD is a CB2 receptor agonist of the endocannabinoid system which, upon activation, inhibits the production of proinflammatory cyto- and chemokines, macrophages and neutrophils/leukocytes of the mucosal immune system. Cannabidiol has antioxidant, analgesic, anti-inflammatory, antitumor, antispastic, anxiolytic, antiemetic, anticonvulsant, neuroprotective, anti-diabetic effect.

Cannabidiol is a lipophilic substance (log P 6-7) with very low solubility in water, prone to degradation, especially in solution by exposure to light, atmospheric oxygen and acidic pH. Cannabidiol bioavailability depends mainly on the level of its absorbtion through the gastrointestinal tract. Lipophilic substances are generally poorly absorbed, inter alia due to their poor solubility and/or dispersibility in water. The oral bioavailability of the active substance also depends on the susceptibility of the substance to the so-called first pass effect. Substances absorbed from the gut must first pass through the liver before distribution to the system, where they are metabolised. It is generally assumed that cannabidiol is largely metabolized in the liver and thus its oral bioavailability is low (S. Zhornitsky, S. Potvin, Pharmaceuticals (2012) 5, 529-552). The formulation containing cannabidiol and the route of administration play a key role in increasing the cannabidiol bioavailability, solubility, chemical stability and residence time in the system.

US 2020/0069606 discloses an analgetic formulation for veterinary use, inter alia in the form of rectal suppositories, which comprises cannabinoids and menthol. The formulation is based on carboxymethyl cellulose and glycerin or other polyalcohol.

US 2018110753 discloses suppositories comprising cannabidiol, Witepsol S58, Ceteareth 35 and isomalt.

### Disclosure of the Invention

The invention provides a pharmaceutical composition containing cannabidiol and hyaluronic acid or a salt thereof with an improved dissolution profile and an improved anti-inflammatory action, the pharmaceutical composition being in the form of suppositories.

These pharmaceutical composition of the invention is intended for use in regeneration, relief, healing of damaged tissues and pain relief, in the treatment of diseases selected from the group of intestinal inflammations, nonspecific intestinal inflammations, vaginal inflammations, endometriosis, prostate inflammations and conditions after surgical removal of prostate, mycoses, cracks and lesions in vaginal and anorectal mucosa, hemorrhoids, proctitis, cryptitis, anal ragad, fissures, perianal fistulas, conditions after proctological surgery, vaginal dryness, vaginal discomfort, vaginal atrophy and dystrophy, painful intercourse, conditions after childbirth, conditions after gynecological surgery, and dystrophy after chemotherapy.

The present invention thus provides a pharmaceutical composition in the form of a suppository, which contains cannabidiol, hyaluronic acid or a salt thereof, and a suppository base containing solid fat, water, and at least one mono(C10-C30)acylglycerol or di(C10-C30)acylglycerol.

Preferably, the pharmaceutical composition contains 0.1 to 5 % w/w of hyaluronic acid or a salt thereof, and 2 to 30 % w/w of cannabidiol.

More preferaby, the pharmaceutical composition contains 0.2 to 0.5 % w/w of hyaluronic acid or a salt thereof, and 3 to 14 % w/w of cannabidiol.

Yet more preferaby, the pharmaceutical composition contains 0.2 to 0.5 % w/w of hyaluronic acid or a salt thereof, and 3 to 6 % w/w of cannabidiol.

The salt of hyaluronic acid may preferably be sodium hyaluronate.

Hyaluronic acid and salts thereof belong among polysaccharides. These substances occur naturally in the body, and they are commonly found in several body fluids, in skin, in several tissues, etc. These substances bind large amounts of water and can provide damaged tissues with a moist environment needed for regeneration and healing, but also for healthy cell division, and to maintain tissue flexibility and elasticity. For the complete release of hyaluronic acid or a salt thereof from the formulation and thus for achieving the maximum possible effect, it is essential that it is dissolved in water, not dispersed in solid form, as is common in other preparations. To this end, it is essential that the suppository base contains water.

Cannabidiol is a phenolic terpenoid, usually obtained by extraction from the plant *Cannabis sativa* L., and separation of cannabidiol from the extract. Cannabidiol is insoluble in water, but dissolves in lipophilic substances. Cannabidiol has antioxidant, anti-inflammatory, regenerative and healing properties.

Within the framework of the present invention, it has been found that cannabidiol and hyaluronic acid or a salt thereof act synergistically in the treatment of diseases and conditions caused by disruption or damage of vaginal, anorectal and/or anal mucosa, including intestinal inflammations, nonspecific intestinal inflammations, vaginal inflammations, endometriosis, prostate inflammations and conditions after surgical removal of prostate, mycoses, cracks and lesions in vaginal and anorectal mucosa, hemorrhoids, proctitis, cryptitis, anal ragad, fissures, perianal fistulas, conditions after proctological surgery, vaginal dryness, vaginal discomfort, vaginal atrophy and dystrophy, painful intercourse, conditions after childbirth, conditions after gynecological surgery, dystrophy after chemotherapy. This combination of active ingredients is very suitable for safe therapeutic use, as it is not irritating and does not show any toxicity at the doses administered.

The suppository base contains at least one mono(C10-C30)acylglycerol or di(C10-C30)acylglycerol, solid fat (adeps solidus) and water.

Preferably, the suppository base contains mono(C10-C30)acylglycerol which is glycerol monostearate.

In a preferred embodiment, the content of water in the pharmaceutical composition is within the range of 5 to 20 % w/w, more preferably 7 to 15 % w/w, even more preferably 9 to 12 % w/w.

Monoacylglycerol(s), solid fat (adeps solidus) and water proved to be a suitable formulation system for suppositories containing cannabidiol and hyaluronic acid or a salt thereof. This formulation system allows for a good homogenization of the actives in the suppository, a good formulation of the suppository, and a good dissolution profile of the actives. At the same time, this formulation system allows for a comfortable, gentle and safe application of suppositories.

In a preferred embodiment, the pharmaceutical composition comprises 0.1 to 5 % w/w of hyaluronic acid or a salt thereof, 2 to 30 % w/w of cannabidiol, 2 to 10 % w/w of glycerol monostearate, 5 to 15 % w/w of water, the balance (to 100 % w/w) being solid fat.

In a more preferred embodiment, the pharmaceutical composition comprises 0.2 to 0.5 % w/w of hyaluronic acid or a salt thereof, 3 to 14 % w/w of cannabidiol, 2 to 10 % w/w of glycerol monostearate, 5 to 15 % w/w of water, the balance (to 100 % w/w) being solid fat.

In a yet more preferred embodiment, the pharmaceutical composition comprises 0.2 to 0.5 % w/w of hyaluronic acid or a salt thereof, 3 to 6 % w/w of cannabidiol, 2 to 5 % w/w of glycerol monostearate, 7 to 12 % w/w of water, the balance (to 100 % w/w) being solid fat.

The suppositories include rectal suppositories and vaginal suppositories.

The weight of one unit dose (one suppository) is 1 to 5 g, preferably 2 to 3 g.

Another aspect of the invention is the pharmaceutical composition according to the present invention for use as a medicament.

Preferably, the pharmaceutical composition of the present invention is for use in regenerating and/or healing damaged tissues, relieving and/or alleviating pain and inflammation, alleviating the symptoms associated with menopause and/or pre-menstrual syndrome.

More specifically, the pharmaceutical composition of the invention is for use in treatment of a disease selected from intestinal inflammations, nonspecific intestinal inflammations, vaginal inflammations, endometriosis, prostate inflammations and conditions after surgical removal of prostate, mycoses, cracks and lesions in vaginal and anorectal mucosa, hemorrhoids, proctitis, cryptitis, anal ragad, fissures, perianal fistulas, conditions after proctological surgery, vaginal dryness, vaginal discomfort, vaginal atrophy and dystrophy, painful intercourse, conditions after childbirth, conditions after gynecological surgery, and dystrophy after chemotherapy.

The pharmaceutical use involves administration of the suppositories to a human or animal subject in need of treatment.

Particularly suitable administration regimens are one suppository daily, two suppositories per day, or one suppository per two days.

### Brief Description of Drawings

Fig. 1: Toxicity of CBD towards the cell line Caco-2.
Fig. 2: Toxicity of CBD towards the cell line Ishikawa.
Fig. 3: Production of IL-6 by the Caco-2 cells following an incubation with LPS.
Fig. 4: Production of IL-8 by the Caco-2 cells following an incubation with LPS.
Fig. 5: Production of IL-6 by the Ishikawa cells following an incubation with LPS.
Fig. 6: Production of IL-8 by the Ishikawa cells following an incubation with LPS.
Fig. 7: Dissolution curves showing the release of sodium hyaluronate from compositions of Examples 1 and 2.

### Examples

### Example 1. Composition and manufacture of suppositories

Unit dose (one suppository) has the following composition:

| | | |
|---|---|---|
| Sodium hyaluronate | 6.6 | mg |
| Cannabidiol (CBD) | 100.0 | mg |
| Glycerol monostearate | 66.0 | mg |
| Solid fat (Adeps solidus) | 1814.0 | mg |
| Water (aqua purificata) | 213.4 | mg |

The suppository base (glycerol monostearate, solid fat and water) is heated to a temperature of about 30 °C, and combined with sodium hyaluronate and cannabidiol which are stirred into the suppository base. The resulting mixture is poured into suppository molds and left to cool down, or alternatively the mixutre is left to cool down and suppositories are then formed from the cold mixture. Rectal or vaginal suppositories can be formed.

### Example 2. Composition of suppositories (comparative example)

Unit dose (one suppository) has the following composition:

| | | |
|---|---|---|
| Sodium hyaluronate | 6.6 | mg |
| Cannabidiol (CBD) | 100.0 | mg |
| Solid fat (Adeps solidus) | 2093.4 | mg |

The manufacturing process is the same as in Example 1, but the suppository base consists only of solid fat.

### Example 3. Assays of cannabidiol, using human intestinal cells (Caco-2) and uterine mucosa cells (Ishikawa)

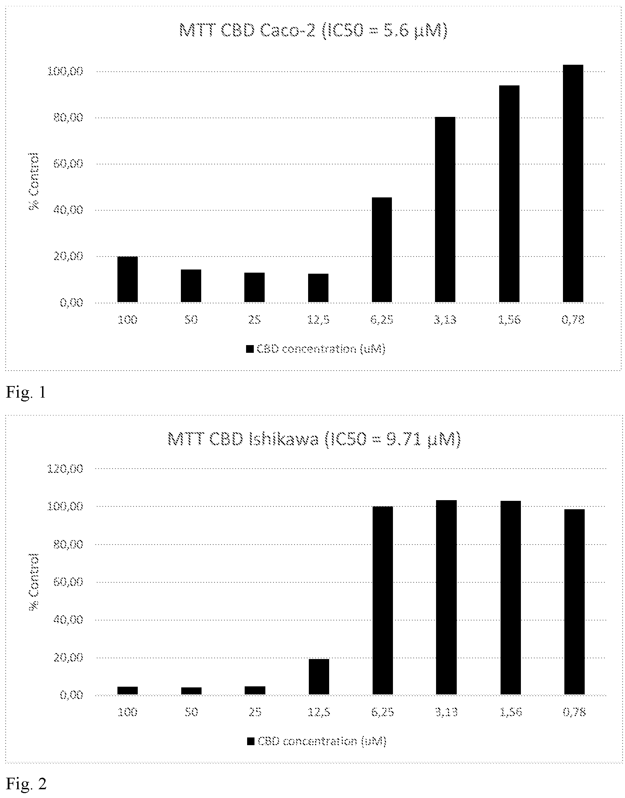

Human Caco-2 intestinal cells and Ishikawa endometrial cells were used in an *in vitro* study of the effect of cannabidiol (CBD) and sodium hyaluronate. In the first phase of the experiment, the safe concentration of CBD was determined based on the results of MTT measurements, a test for the reduction of 3- (4,5-dimethylthiazol-2-yl)-2,5-diphenyltetrazolium bromide (MTT) to the corresponding formazan. The test was performed according to M.D. Maines, L.G. Costa, D.J. Reed, et al. Current protocols in toxicology. New York: John Wiley & Sons. (1998). The test results are shown in Figures 1 and 2.

Cells seeded in a 96-well plate at a concentration of 3 x 10⁴ cells/ml, 100 µl/well, which were allowed to adhere for 24 hours, were subsequently incubated for 24 hours with CBD in a concentration range of 100 to 0.78 µmol/l dissolved in serum-free culture medium containing MEM (Minimum Essential Medium Eagle) suplemented by 2mM glutamine, 1% NEAA (Non essential Amino Acid), and penicillin 100 IU/ml and streptomycin 100 ug/ml. At the end of the incubation period, 100 µl of MTT mixture (0.5 mg/mL in serum-free culture medium) was added for the period of 2 hours. At the end of the incubation period and aspiration of the MTT mixture, 100 µl of DMSO with NH₃ (1%, v/v) were added. The absorbance was measured at 540 nm (INFINITE M200 PRO Tecan, Schoeller). The results are plotted as a percentage of control (cells cultured only with serum-free culture medium). The assay was performed in triplicates.

Based on the MTT values, two sub-toxic concentrations of CBD (3 and 5 µmol/l) were selected and used to determine its anti-inflammatory effect on human intestinal (Caco-2) and endometrial (Ishikawa) cells.

Cells (Caco-2, Ishikawa) were seeded in 6-well plates in culture medium containing MEM (Minimum Essential Medium Eagle) suplemented by 2mM glutamine, 1% NEAA (Non essential Amino Acid), 10 % FBS (Fetal Bovine Serum), penicillin 100 IU/ml and streptomycin 100 ug/ml and allowed to adhere for a minimum of 24 hours and then allowed to grow to confluence. They were then rinsed with PBS (2 ml) and treated with LPS (*E. coli*) 2 µg/ml in serum-free culture medium, 2 ml per well 3 times after 3 hours. After the last 3 hours of incubation, the test substances (CBD, CBD + HA in serum-free culture medium) were added to the cells and the samples were incubated for another 22 hours. The culture medium was then removed, frozen at -80 °C for downstream determination of pro-inflammatory cytokines (IL-6 and IL-8) by ELISA (Human ELISA Development Kit, Cat. Nos. 900-K16 and 900-K18, PeproTech, Baria, Czech Republic). The results are expressed relative to a control K (cells cultured only with serum-free culture medium), as a percentage of the control, expressed as mean ± SEM. The measurement was performed in three independent experiments. KL are cells cultured with LPS. Testing was performed in triplicates.

Cytokines play a key role in the pathogenesis of inflammatory diseases, and IL-6 has been shown to be the most important one. IL-6 is involved in the development of idiopathic intestinal inflammations, such as Crohn's disease or ulcerative colitis (Involvement of IL-6 in the Pathogenesis of Inflammatory Bowel Disease and Colon Cancer, R. Atreya, M. F. Neurath, Clin. Rev. Allergy Immunol. 2005, 28, 187) and it is a clinically relevant parameter which correlates with inflammatory activity. The same is true for IL-6 and endometriosis (An increased level of IL-6 suppresses NK cell activity in peritoneal fluid of patients with endometriosis via regulation of SHP-2 expression, Y.J. Kang, I.C. Jeung, A. Park, Y.J. Park, H. Jung, T.D. Kim, H.G. Lee, I. Choi, S.R. Yoon. Hum. Reprod. 2014, 29, 2176). It has also been found that another cytokine whose concentration in the peritoneal fluid of women with endometriosis is increased and its level correlates with the severity of the disease is IL-8. IL-8 is also assimed to play a role in affecting the growth and maintenance of ectopic endometrial tissue not only by chemoattraction but also by directly affecting endometrial cell proliferation (Local Cytokines in Endometrial Tissue: The Role of Interleukin-8 in the Pathogenesis of Endometriosis. A. Arici, Ann. N. Y. Acad. Sci. 2002, 955, 101.)

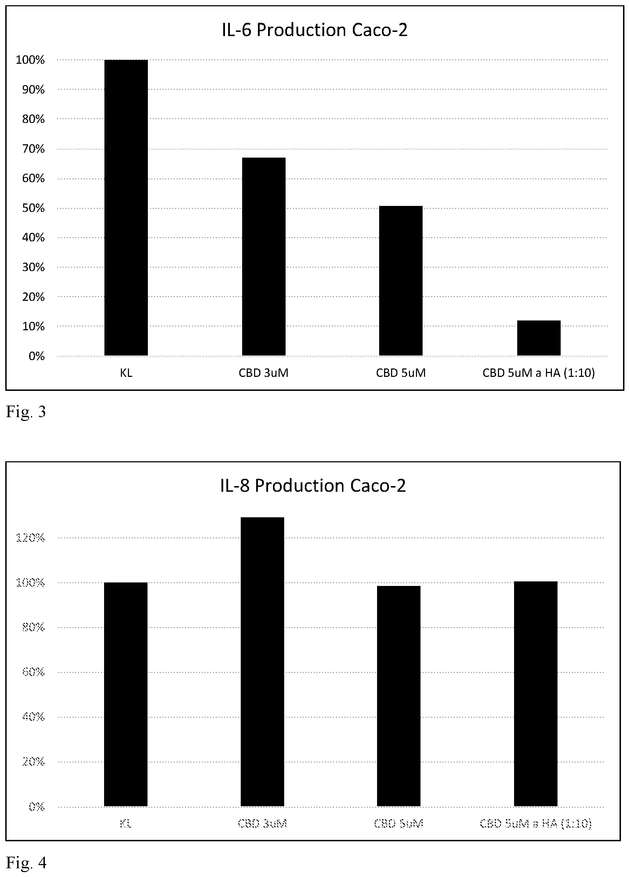

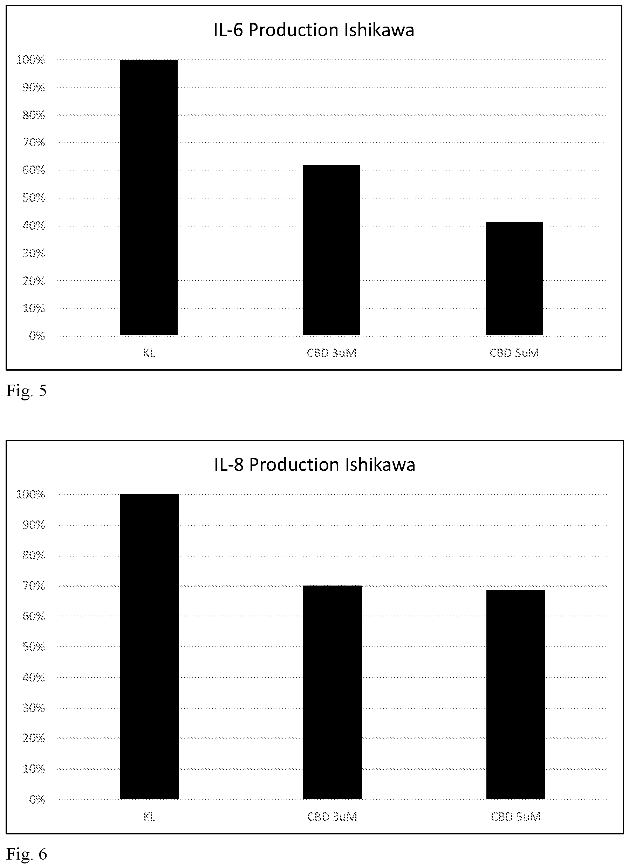

In an experiment after induction of inflammation in human intestinal cells (Caco-2) and endometrial cells (Ishikawa) by LPS and subsequent administration of both doses of cannabidiol, a significant decrease in IL-6 levels was found in both cell models (Figure 3 and 5). Furthermore, in the case of Caco-2 cells, a synergistic effect of the CBD: HA combination on reducing the concentration of IL-6 was found (Figure 3). For IL-8 and Caco-2 cells (Figure 4), its concentration is at the same level using a higher dose of CBD. In Ishikawa endometrial cells, IL-8 levels again decrease for both doses of CBD used. However, our observations contradicts the article Cannabidiol and Palmitoylethanolamide are anti-inflammatory in the acutely inflamed human colon. D. G. Couch, Ch. Tasker, E. Theophilidou, J. Lund, S. E. O'Sullivan, Clin. Sci. (Lond.) 2017, 131, 2611, the authors of which observed the opposite effect in the case of the IL-6 and Caco-2 model, i.e. an increase in its concentration by more than 70% compared to the original value after induction of inflammation. In conclusion, our experiments showed the anti-inflammatory effect of both doses of cannabidiol, demonstrably in the longer time perspective of 24-h incubation. In addition, a synergistic effect of CBD with sodium hyaluronate was demonstrated for Caco-2 cells and IL-6.

### Example 4: Dissolution test - release of sodium hyaluronate

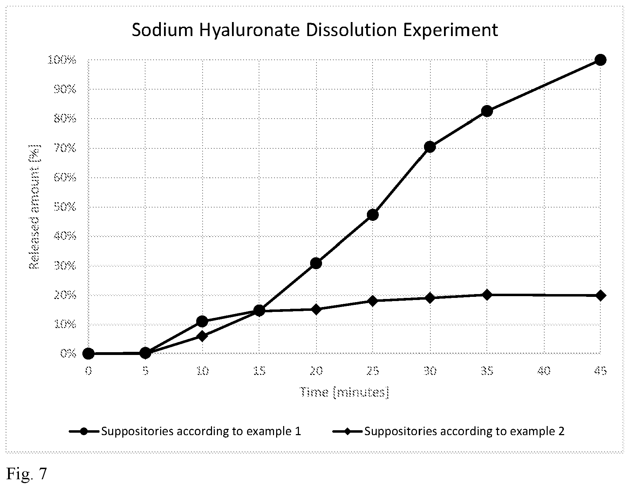

A basket dissolution method was used to determine the release of sodium hyaluronate from the formulations of Examples 1 and 2, corresponding to a dose of 6.6 mg/unit dose. The medium temperature was 37 ± 0.5 °C, the basket speed was 100/min. The suppository was placed in a basket and placed in a container holding phosphate buffer pH 7.0. Samples were taken at 5, 10, 15, 20, 25, 30, 35 and 45 minutes. The volume removed was compensated by the addition of the buffer. The dissolution curves are shown in Fig. 7. The rate of release of sodium hyaluronate from the formulation of Example 1 is significantly faster, starting from 15 minutes, compared to the formulation of Example 2. After 45 minutes, all of the sodium hyaluronate was released from Formulation 1, while from Formulation 2 only 20% of sodium hyaluronate was released. The results show that for an effective release of sodium hyaluronate, it is essential that the formulation contains water (see Example 1). Sodium hyaluronate only dispersed in a lipophilic medium does not show suitable dissolution properties (see Example 2).

### Example 5: Skin irritability assay

The test was performed in accordance with ČSN EN ISO 10993-10: 2010, Part 10. Samples of suppositories prepared according to Example 1 were tested. 30 human volunteers were tested. 50 mg of the test material on a 1.8 cm diameter gauze disc of a closed chamber (IQ Ultra Patch Test Chembers, Chemotechnical Diagnostics Sweden) is applied to the upper outer part of the arm moistened with water. The prescribed exposure time is kept. After removing the pad and evaluating the test, the skin is rinsed and wiped under running water. Clinical observation and classification is performed immediately after pad removal, and at 2 h, 24 h, 48 h and 72 h intervals after pad removal.

Furthermore, a positive control of 20% sodium lauryl sulfate (wt.%) in an amount of 0.4 ml on a 1.8 cm diameter gauze disc of a closed chamber (IQ Ultra Patch Test Chembers, Chemotechnical Diagnostics Sweden) is applied to the outer arm moistened with water. The prescribed exposure time is used. After removing the pad and evaluating the test, the skin is rinsed and wiped under running water. Clinical observation and classification is performed immediately after pad removal, and at 2 h, 24 h, 48 h and 72 h intervals after pad removal.

The reaction is classified by the numbers 0 - no reaction; 1 - weakly positive reaction; 2 - slightly positive reaction; 3 - strongly positive reaction.

The results are shown in Tables 1 and 2.

0 out of 30 volunteers showed any adverse skin reaction to the test product. 30 out of 30 volunteers showed a skin reaction to the positive control.

**Table 1: Skin irritability assay - test suppositories**

| **Volunteer** | | | Reading interval | | | | | |
|---|---|---|---|---|---|---|---|---|
| | | | EXPOSITION TIME: 15min / 30min / 1h / 2h / 3h / 4h | | | | | |
| | | | **Classification of skin reaction after:** | | | | | **Reaction** |
| **Order** | **Age** | **Sex** | **0 h** | **2h** | **24 h** | **48 h** | **72 h** | **yes / no** |
| 1 | 45 | F | 000000 | 000000 | 000000 | 000000 | 000000 | **NO** |
| 2 | 40 | F | 000000 | 000000 | 000000 | 000000 | 000000 | **NO** |
| 3 | 39 | F | 000000 | 000000 | 000000 | 000000 | 000000 | **NO** |
| 4 | 40 | F | 000000 | 000000 | 000000 | 000000 | 000000 | **NO** |
| 5 | 54 | F | 000000 | 000000 | 000000 | 000000 | 000000 | **NO** |
| 6 | 42 | F | 000000 | 000000 | 000000 | 000000 | 000000 | **NO** |
| 7 | 43 | F | 000000 | 000000 | 000000 | 000000 | 000000 | **NO** |
| 8 | 50 | F | 000000 | 000000 | 000000 | 000000 | 000000 | **NO** |
| 9 | 44 | F | 000000 | 000000 | 000000 | 000000 | 000000 | **NO** |
| 10 | 47 | F | 000000 | 000000 | 000000 | 000000 | 000000 | **NO** |
| 11 | 53 | F | 000000 | 000000 | 000000 | 000000 | 000000 | **NO** |
| 12 | 37 | F | 000000 | 000000 | 000000 | 000000 | 000000 | **NO** |
| 13 | 34 | F | 000000 | 000000 | 000000 | 000000 | 000000 | **NO** |
| 14 | 27 | F | 000000 | 000000 | 000000 | 000000 | 000000 | **NO** |
| 15 | 44 | F | 000000 | 000000 | 000000 | 000000 | 000000 | **NO** |
| 16 | 35 | F | 000000 | 000000 | 000000 | 000000 | 000000 | **NO** |
| 17 | 47 | F | 000000 | 000000 | 000000 | 000000 | 000000 | **NO** |
| 18 | 49 | F | 000000 | 000000 | 000000 | 000000 | 000000 | **NO** |
| 19 | 69 | F | 000000 | 000000 | 000000 | 000000 | 000000 | **NO** |
| 20 | 45 | F | 000000 | 000000 | 000000 | 000000 | 000000 | **NO** |
| 21 | 42 | M | 000000 | 000000 | 000000 | 000000 | 000000 | **NO** |
| 22 | 46 | M | 000000 | 000000 | 000000 | 000000 | 000000 | **NO** |
| 23 | 42 | M | 000000 | 000000 | 000000 | 000000 | 000000 | **NO** |
| 24 | 45 | M | 000000 | 000000 | 000000 | 000000 | 000000 | **NO** |
| 25 | 54 | M | 000000 | 000000 | 000000 | 000000 | 000000 | **NO** |
| 26 | 54 | M | 000000 | 000000 | 000000 | 000000 | 000000 | **NO** |
| 27 | 51 | M | 000000 | 000000 | 000000 | 000000 | 000000 | **NO** |
| 28 | 36 | M | 000000 | 000000 | 000000 | 000000 | 000000 | **NO** |
| 29 | 49 | M | 000000 | 000000 | 000000 | 000000 | 000000 | **NO** |
| 30 | 29 | M | 000000 | 000000 | 000000 | 000000 | 000000 | **NO** |
| Total number of reactions | | | 000000 | 000000 | 000000 | 000000 | 000000 | **30/30** |

**Table 2: Skin irritability assay - positive control**

| Volunteer | | | Reading interval | | | | | |
|---|---|---|---|---|---|---|---|---|
| | | | EXPOSITION TIME: 15min / 30min / 1h / 2h / 3h / 4h | | | | | |
| | | | **Classification of skin reaction after:** | | | | | **Reaction** |
| **Ord er** | **Ag e** | **Sex** | **0 h** | **2 h** | **24 h** | **48 h** | **72 h** | **yes / no** |
| 1 | 45 | F | 001111 | 000111 | 000011 | 000001 | 000000 | **YES** |
| 2 | 40 | F | 000001 | 000001 | 000000 | 000000 | 000000 | **YES** |
| 3 | 39 | F | 00112 | 000111 | 000011 | 000000 | 000000 | **YES** |
| 4 | 40 | F | 000112 | 001222 | 001122 | 000111 | 000011 | **YES** |
| 5 | 54 | F | 000111 | 000012 | 000011 | 000011 | 000000 | **YES** |
| 6 | 42 | F | 000011 | 000112 | 000011 | 000001 | 000000 | **YES** |
| 7 | 43 | F | 000001 | 000111 | 000111 | 000001 | 000000 | **YES** |
| 8 | 50 | F | 000011 | 000012 | 000001 | 000000 | 000000 | **YES** |
| 9 | 44 | F | 000001 | 000111 | 000111 | 000001 | 000000 | **YES** |
| 10 | 47 | F | 011122 | 001112 | 001112 | 000011 | 000001 | **YES** |
| 11 | 53 | F | 000011 | 000011 | 000001 | 000000 | 000000 | **YES** |
| 12 | 37 | F | 000000 | 000001 | 000000 | 000000 | 000000 | **YES** |
| 13 | 34 | F | 001111 | 011111 | 000111 | 000011 | 000000 | **YES** |
| 14 | 27 | F | 000112 | 000111 | 000011 | 000001 | 000000 | **YES** |
| 15 | 44 | F | 000012 | 000111 | 000011 | 000001 | 000000 | **YES** |
| 16 | 35 | F | 001111 | 000011 | 000001 | 000000 | 000000 | **YES** |
| 17 | 47 | F | 000122 | 000112 | 000111 | 000011 | 000001 | **YES** |
| 18 | 49 | F | 000011 | 000011 | 000001 | 000000 | 000000 | **YES** |
| 19 | 69 | F | 000001 | 001111 | 000111 | 000001 | 000001 | **YES** |
| 20 | 45 | F | 000112 | 000111 | 000011 | 000000 | 000000 | **YES** |
| 21 | 42 | M | 000001 | 000012 | 000011 | 000001 | 000000 | **YES** |
| 22 | 46 | M | 011111 | 011112 | 000111 | 000011 | 000001 | **YES** |
| 23 | 42 | M | 000011 | 000011 | 000001 | 000000 | 000000 | **YES** |
| 24 | 45 | M | 000112 | 001111 | 000011 | 000001 | 000000 | **YES** |
| 25 | 54 | M | 000011 | 000111 | 000111 | 000011 | 000000 | **YES** |
| 26 | 54 | M | 000111 | 001222 | 000112 | 000001 | 000001 | **YES** |
| 27 | 51 | M | 000011 | 000011 | 000001 | 000000 | 000000 | **YES** |
| 28 | 36 | M | 000011 | 000111 | 000001 | 000000 | 000000 | **YES** |
| 29 | 49 | M | 001112 | 000111 | 000011 | 000001 | 000000 | **YES** |
| 30 | 29 | M | 000001 | 000001 | 000000 | 000000 | 000000 | **YES** |
| Total number of reactions | | | 0/2/6/14/2 3/29 | 0/2/7/19/2 7/30 | 0/0/2/10/2 0/27 | 0/0/0/1/7 /18 | 0/0/0/0/ 1/6 | **30/30** |

### Example 6: Cytotoxicity assay

Balb/c 3T3-L1 mouse fibroblasts (ECACC 86052701) were used in the cytotoxicity assay. The cells were cultured in D-MEM medium (Dulbecco's modification of Eagle's essential essential medium, Lonza) containing antibiotics (penicillin 100 IU/ml, streptomycin 100 ug/ml), enriched with 10% inactivated calf serum, pH 7.2.

Suppositories prepared according to Example 1 were tested. The positive control (PK) was sodium lauryl sulfate (SLS), the cell control was serum-free culture medium.

The suppository extract was prepared in the ratio of 0.2 g of sample per 1 ml of extracting agent (serum-free D-MEM) according to ČSN EN ISO 10993-12 (2012). The extraction was performed for 24 hours at 37 °C, in a closed culture flask under aseptic conditions according to ČSN EN ISO 10993-5 (2012). The resulting 100% extract was further diluted with serum-free culture medium.

Flat bottom tissue culture plates of 8x12 wells were used to culture the cells. The cells were suspended at a concentration of 10⁵ cells in 1 ml of the culture medium, inoculating 0.1 ml of the suspension into 1 well. Culture conditions 37 °C, 7.5% CO₂. Pre-cultivation to obtain a cell culture monolayer was performed 24 hours before exposure to the test material. The cultures were established in quadruplets for the test extracts and for the controls. After 24 hours of precultivation, the culture medium and 0.2 ml of the extract from the test sample, or its serial dilution, respectively. This was followed by culture for 24 hours at 37 °C, 7.5% CO₂ and staining with neutral red (0.2 ml of neutral red solution per well, incubation for 3 hours, ethanol/acetic acid fixation solution). After completion of the culture, cytotoxicity was determined quantitatively (fluorimetrically) based on the incorporation of neutral red as a vital dye.

The fluorimetric method for determining cytotoxicity is based on the incorporation of a vital dye into living cells and the detection of fluorescence in an excitation (530 nm) and emission filter (590 nm) system using cold light. The degree of cytotoxicity is expressed in % of the detected fluorescence in the culture containing the test extract relative to the control culture in the absence of the test extract. Fluorescence is detected in FSU units of fluorescence intensity. For quantification, the viability of the culture is calculated as follows: culture viability (%) = (average FSU of the test sample - average FSU of the blank) / (average FSU of the control - average FSU of the blank)

The mean fluorescence values for each group represent the average fluorescence value from all four wells after subtracting the average fluorescence value of the eight wells with fixation solution (blank). The control is serum-free culture medium.

The test was repeated twice. The data from both repetitions are in the following tables:

### Test 1

| **Sample Dilution** | **Mean fluorescence (FSU)** | **Relative to control %** |
|---|---|---|
| **VZ 3/19/182** | | |
| **10%** | 2894.9 | **104.3** |
| **25%** | 2927.9 | **105.5** |
| **50%** | 2227.9 | **80.3** |
| **100%** | 1757.9 | **63.3** |

| **PK - SLS** | | |
|---|---|---|
| 1 µg/ml | 2743.4 | 98.8 |
| 10 µg/ml | 891.1 | 32.1 |
| 20 µg/ml | 113.9 | 16.0 |

### Test 2

| **Sample Dilution** | **Mean fluorescence (FSU)** | **Relative to control %** |
|---|---|---|
| **VZ 3/19/182** | | |
| **10%** | 3558.8 | **110.4** |
| **25%** | 3459.8 | **107.4** |
| **50%** | 3465.3 | **107.5** |
| **100%** | 2046.8 | **63.5** |

| **PK** - **SLS** | | |
|---|---|---|
| 1 µg/ml | 3187.8 | 98.9 |
| 10 µg/ml | 542.5 | 16.8 |
| 20 µg/ml | 326.3 | 10.1 |

At the maximum concentration of the extract and the contact time of 24 hours, a slight degree of cytotoxicity was found in the mouse cell line *in vitro,* at further dilutions the extract was completely tolerated by the cell culture. Due to the longer contact time compared to the usual contact time on use of suppositories, use on free cells and elimination of gradual release (cells were grown directly in the complete extract), the mild *in vitro* cytotoxicity of the composition can be considered safe, with regard to other positive tests. Furthermore, it is assumed that the cytotoxicity in this assay is at least partly due to the lipophilic medium.

## Claims

1. Pharmaceutical composition in the form of suppository, containing cannabidiol, hyaluronic acid or a salt thereof, and a suppository base containing solid fat, water, and at least one mono(C10-C30)acylglycerol or di(C10-C30)acylglycerol.

2. Pharmaceutical composition according to claim 1, which contains 0.1 to 5 % w/w of hyaluronic acid or a salt thereof, and 2 to 30 % w/w of cannabidiol.

3. Pharmaceutical composition according to claim 1, which contains 0.2 to 0.5 % w/w of hyaluronic acid or a salt thereof, and 3 to 14 % w/w of cannabidiol.

4. Pharmaceutical composition according to claim 1, which contains 0.2 to 0.5 % w/w of hyaluronic acid or a salt thereof, and 3 to 6 % w/w of cannabidiol.

5. Pharmaceutical composition according to any one of the preceding claims, wherein the salt of hyaluronic acid is sodium hyaluronate.

6. Pharmaceutical composition according to any one of the preceding claims, which contains 5 to 20 % w/w, preferably 7 to 15 % w/w of water.

7. Pharmaceutical composition according to claim 1, wherein the suppository base contains solid fat, water, and glycerol monostearate.

8. Pharmaceutical composition according to claim 7, which contains 0.1 to 5 % w/w of hyaluronic acid or a salt thereof, 2 to 30 % w/w of cannabidiol, 2 to 10 % w/w of glycerol monostearate, 5 to 15 % w/w of water, the balance being solid fat.

9. Pharmaceutical composition according to claim 7, which contains 0.2 to 0.5 % w/w of hyaluronic acid or a salt thereof, 3 to 14 % w/w of cannabidiol, 2 to 10 % w/w of glycerol monostearate, 5 to 15 % w/w of water, the balance being solid fat.

10. Pharmaceutical composition according to claim 7, which contains 0.2 to 0.5 % w/w of hyaluronic acid or a salt thereof, 3 to 6 % w/w of cannabidiol, 2 to 5 % w/w of glycerol monostearate, 7 to 12 % w/w of water, the balance being solid fat.

11. Pharmaceutical composition according to any one of claims 1 to 10 for use as a medicament.

12. Pharmaceutical composition according to any one of claims 1 to 10 for use in regenerating and/or healing damaged tissues, or in relieving and/or alleviating pain and inflammation, or in alleviating the symptoms associated with menopause and/or pre-menstrual syndrome.

13. Pharmaceutical composition according to any one of claims 1 to 10 for use in the treatment of a disease selected from intestinal inflammations, nonspecific intestinal inflammations, vaginal inflammations, endometriosis, prostate inflammations and conditions after surgical removal of prostate, mycoses, cracks and lesions in vaginal and anorectal mucosa, hemorrhoids, proctitis, cryptitis, anal ragad, fissures, perianal fistulas, conditions after proctological surgery, vaginal dryness, vaginal discomfort, vaginal atrophy and dystrophy, painful intercourse, conditions after childbirth, conditions after gynecological surgery, and dystrophy after chemotherapy; in prevention and/or treatment of radiation-induced skin/tissue reactions; or in preparing the endometrium to be receptive after replacing one or more embryos into the uterine cavity in assisted reproductive technologies.

14. Pharmaceutical composition for use according to any one of claims 11 to 13, wherein the use includes an administration regimen selected from one suppository daily, two suppositories per day, and one suppository per two days.

## Patentansprüche

1. Pharmazeutische Zusammensetzung in Form eines Zäpfchens, enthaltend Cannabidiol, Hyaluronsäure oder ein Salz davon, und eine Zäpfchengrundlage, die festes Fett, Wasser und mindestens ein Mono(C10-C30)acylglycerol oder Di(C10-C30)acylglycerol enthält.

2. Pharmazeutische Zusammensetzung nach Anspruch 1, die 0,1 bis 5 Gew.-% Hyaluronsäure oder ein Salz davon und 2 bis 30 Gew.-% Cannabidiol enthält.

3. Pharmazeutische Zusammensetzung nach Anspruch 1, die 0,2 bis 0,5 Gew.-% Hyaluronsäure oder ein Salz davon und 3 bis 14 Gew.-% Cannabidiol enthält.

4. Pharmazeutische Zusammensetzung nach Anspruch 1, die 0,2 bis 0,5 Gew.-% Hyaluronsäure oder ein Salz davon und 3 bis 6 Gew.-% Cannabidiol enthält.

5. Pharmazeutische Zusammensetzung nach einem der vorhergehenden Ansprüche, wobei das Salz der Hyaluronsäure Natriumhyaluronat ist.

6. Pharmazeutische Zusammensetzung nach einem der vorhergehenden Ansprüche, die 5 bis 20 Gew.-%, vorzugsweise 7 bis 15 Gew.-% Wasser enthält.

7. Pharmazeutische Zusammensetzung nach Anspruch 1, wobei die Zäpfchengrundlage festes Fett, Wasser und Glycerinmonostearat enthält.

8. Pharmazeutische Zusammensetzung nach Anspruch 7, die 0,1 bis 5 Gew.-% Hyaluronsäure oder ein Salz davon, 2 bis 30 Gew.-% Cannabidiol, 2 bis 10 Gew.-% Glycerinmonostearat, 5 bis 15 Gew.-% Wasser enthält, wobei der Rest festes Fett ist.

9. Pharmazeutische Zusammensetzung nach Anspruch 7, die 0,2 bis 0,5 Gew.-% Hyaluronsäure oder ein Salz davon, 3 bis 14 Gew.-% Cannabidiol, 2 bis 10 Gew.-% Glycerinmonostearat, 5 bis 15 Gew.-% Wasser enthält, wobei der Rest festes Fett ist.

10. Pharmazeutische Zusammensetzung nach Anspruch 7, die 0,2 bis 0,5 Gew.-% Hyaluronsäure oder ein Salz davon, 3 bis 6 Gew.-% Cannabidiol, 2 bis 5 Gew.-% Glycerinmonostearat, 7 bis 12 Gew.-% Wasser enthält, wobei der Rest festes Fett ist.

11. Pharmazeutische Zusammensetzung nach einem der Ansprüche 1 bis 10 zur Verwendung als Arzneimittel.

12. Pharmazeutische Zusammensetzung nach einem der Ansprüche 1 bis 10 zur Verwendung bei der Regeneration und/oder Heilung geschädigten Gewebes oder bei der Linderung und/oder Reduktion von Schmerzen und Entzündungen oder bei der Linderung der mit der Menopause und/oder dem prämenstruellen Syndrom verbundenen Symptome.

13. Pharmazeutische Zusammensetzung nach einem der Ansprüche 1 bis 10 zur Verwendung bei der Behandlung einer Krankheit, ausgewählt aus Darmentzündungen, unspezifischen Darmentzündungen, Vaginalentzündungen, Endometriose, Prostataentzündungen und Zuständen nach chirurgischer Entfernung der Prostata, Mykosen, Rissen und Läsionen in der Vaginal- und Anorektalschleimhaut, Hämorrhoiden, Proktitis, Kryptitis, Analragad, Fissuren, perianalen Fisteln, Zuständen nach proktologischen Operationen, vaginaler Trockenheit, vaginalen Beschwerden, Vaginalatrophie und -dystrophie, schmerzhaftem Geschlechtsverkehr, Zuständen nach der Geburt, Zuständen nach gynäkologischen Operationen und Dystrophie nach Chemotherapie; zur Vorbeugung und/oder Behandlung strahleninduzierter Haut-/Gewebereaktionen; oder zur Vorbereitung des Endometriums auf die Aufnahme nach dem Einsetzen eines oder mehrerer Embryonen in die Gebärmutterhöhle bei assistierten Reproduktionstechnologien.

14. Pharmazeutische Zusammensetzung zur Verwendung nach einem der Ansprüche 11 bis 13, wobei die Verwendung ein Verabreichungsschema umfasst, das ausgewählt ist aus einem Zäpfchen täglich, zwei Zäpfchen pro Tag und einem Zäpfchen alle zwei Tage.

## Revendications

1. Composition pharmaceutique sous forme de suppositoire, contenant du cannabidiol, de l'acide hyaluronique ou un sel de celui-ci, et une base de suppositoire contenant de la matière grasse solide, de l'eau et au moins un mono(C10-C30)acylglycérol ou di(C10-C30)acylglycérol.

2. Composition pharmaceutique selon la revendication 1, qui contient 0,1 à 5 % p/p d'acide hyaluronique ou un sel de celui-ci, et 2 à 30 % p/p de cannabidiol.

3. Composition pharmaceutique selon la revendication 1, qui contient 0,2 à 0,5 % p/p d'acide hyaluronique ou un sel de celui-ci, et 3 à 14 % p/p de cannabidiol.

4. Composition pharmaceutique selon la revendication 1, qui contient 0,2 à 0,5 % p/p d'acide hyaluronique ou d'un sel de celui-ci, et 3 à 6 % p/p de cannabidiol.

5. Composition pharmaceutique selon l'une quelconque des revendications précédentes, dans laquelle le sel d'acide hyaluronique est l'hyaluronate de sodium.

6. Composition pharmaceutique selon l'une quelconque des revendications précédentes, qui contient 5 à 20 % p/p, de préférence 7 à 15 % p/p d'eau.

7. Composition pharmaceutique selon la revendication 1, où la base du suppositoire contient de la matière grasse solide, de l'eau et du monostéarate de glycérol.

8. Composition pharmaceutique selon la revendication 7, qui contient 0,1 à 5 % p/p d'acide hyaluronique ou d'un sel de celui-ci, 2 à 30 % p/p de cannabidiol, 2 à 10 % p/p de monostéarate de glycérol, 5 à 15 % p/p d'eau, le reste étant de la matière grasse solide.

9. Composition pharmaceutique selon la revendication 7, qui contient 0,2 à 0,5 % p/p d'acide hyaluronique ou d'un sel de celui-ci, 3 à 14 % p/p de cannabidiol, 2 à 10 % p/p de monostéarate de glycérol, 5 à 15 % p/p d'eau, le reste étant de la matière grasse solide.

10. Composition pharmaceutique selon la revendication 7, qui contient 0,2 à 0,5 % p/p d'acide hyaluronique ou d'un sel de celui-ci, 3 à 6 % p/p de cannabidiol, 2 à 5 % p/p de monostéarate de glycérol, 7 à 12 % p/p d'eau, le reste étant de la matière grasse solide.

11. Composition pharmaceutique selon l'une quelconque des revendications 1 à 10 pour utilisation comme médicament.

12. Composition pharmaceutique selon l'une quelconque des revendications 1 à 10 pour utilisation pour régénérer et/ou cicatriser des tissus endommagés, ou pour soulager et/ou atténuer la douleur et l'inflammation, ou pour atténuer les symptômes associés à la ménopause et/ou au syndrome prémenstruel.

13. Composition pharmaceutique selon l'une quelconque des revendications 1 à 10, pour utilisation dans le traitement d'une maladie choisie parmi les inflammations intestinales, les inflammations intestinales non spécifiques, les inflammations vaginales, l'endométriose, les inflammations de la prostate et les affections après ablation chirurgicale de la prostate, les mycoses, les fissures et les lésions de la muqueuse vaginale et anorectale, les hémorroïdes, la rectite, la cryptite, la ragade anale, les fissures, les fistules périanales, les affections après chirurgie proctologique, la sécheresse vaginale, l'inconfort vaginal, l'atrophie et la dystrophie vaginales, les rapports sexuels douloureux, les affections après l'accouchement, les affections après chirurgie gynécologique et la dystrophie après chimiothérapie; dans la prévention et/ou le traitement des réactions cutanées/tissulaires induites par les radiations; ou dans la préparation de l'endomètre à être réceptif après le remplacement d'un ou plusieurs embryons dans la cavité utérine dans les technologies de procréation assistée.

14. Composition pharmaceutique pour utilisation selon l'une quelconque des revendications 11 à 13, où l'utilisation comprend un régime d'administration choisi parmi un suppositoire par jour, deux suppositoires par jour et un suppositoire tous les deux jours.
